# EUROPEAN PATENT APPLICATION

(11) **EP 0 692 540 A2**
(43) Date of publication of application: **17.01.1996**
(21) Application number: 95109002.6
(22) Date of filing: 12.06.1995
(51) Int. Cl.: C12Q 1/68

(54) **Oligonucleotide primers and probes for detection of bacteria**

(30) Priority: 17.06.1994 US 261608
(71) Applicant: Becton Dickinson and Company, Franklin Lakes, New Jersey 07417-1880 (US)
(72) Inventor: Meyer, Mary Kathryn, Durham, NC 27707 (US)
(74) Representative: Rambelli, Paolo

(57) **Abstract**

The present invention relates to oligonucleotide primers for the amplification of a nucleic acid target sequence which is characteristic of eubacteria. The invention also relates to an oligonucleotide probe which may be used in the detection of the nucleic acid target sequence. The primers and probe may also be included in a kit.

## Description

### BACKGROUND OF THE INVENTION

### Field of the Invention

The present invention broadly relates to the amplification and subsequent detection of nucleic acid sequences characteristic of eubacteria.

### Background Art

Septicemia is a systemic disease associated with the presence and persistence of pathogenic microorganisms or their toxins in the blood. Sepsis is the 13th leading cause of death in the United States and the most common cause of death in surgical intensive care units. There has been a 140% increase in the incidence of septicemia since 1980 due to the aging population, use of intravascular devices and immuno-compromised patients.

The current method for detection of septicemia relies on the cultivation of organisms from a clinical sample. Blood is drawn from patients, inoculated into culture medium and monitored for 1 to 7 days for detectable growth. Additionally, the clinical sample is often evaluated by other means of detection which include gram-staining, biochemical assays and pure culture isolation for microscopic morphology. These diagnostic methods suffer from false negatives due to a low cell count in a clinical sample. Furthermore, the disease is difficult to treat. Often therapeutic intervention begins after the unchecked inflammatory response has begun to destroy tissue, leading to organ failure and eventually death of the patient. Thus, a reliable diagnostic for septic shock, with a shorter time to results, could potentially increase the efficacy of therapeutics and improve patient prognosis.

Thus, numerous nucleic acid sequences which correspond to conserved regions of bacterial 16S rRNA have been identified and described as oligonucleotide primers and probes which may be useful in the detection and identification of bacterial organisms which contribute to sepsis or to determine whether therapeutic intervention has improved a patient's prognosis. For example, probes for screening to determine the effectiveness of a lysis procedure on bacteria are reported by Jones, C.L et al., Analytical Biochemistry 181, 23 (1989). A "panprobe" was developed by aligning 16S rRNA sequences from twelve genetically unrelated eubacterial species to identify a highly conserved sequence.

Universal probes for detection of bacteria and primers for amplification of bacterial nucleic acid sequences are also disclosed in EPA-0 479 117 A1. Two universal probes are disclosed which correspond to a highly conserved region in the 16S rRNA gene. Also disclosed are two universal bacterial primers.

Universal bacterial probes to 16S rRNA are also disclosed in PCT Publication WO 90/15157. This publication identifies seven bacterial probes.

Another bacterial nucleic acid probe which is complementary to a highly conserved region of the 16S rRNA of bacteria is disclosed in PCT Publication No. WO 91/00926. The probe is used to detect 16S rRNA in a sample and by comparison with known standards used to estimate the total bacterial count in the sample.

Five more bacterial DNA probes are disclosed in PCT Publication No. WO 90/01560.

Because the present nucleic amplification system of choice in the act is the polymerase chain reaction (PCR) (U.S. Pat. No. 4,683,202), the primers described above are utilized to amplify target nucleic acid sequences greater than fifty (50) base pairs in length (i.e. the primers hybridize to the target sequence more than 50 base pairs from one another). Also, the above-described probes therefore detect target sequences of greater than 50 base pairs. However, shorter target sequences (i.e. 50 base pairs or less) are more advantageous due to more specific hybridization with probes and usefulness in other amplification systems such as Strand Displacement Amplification (SDA) (see Walker G.T. et al., Proc Nat'l. Acad. Sci. USA 89, 392(1992)) which at present are not as efficient at amplifying targets greater than 50 base pairs, but are very efficient with targets of 50 base pairs or less.

### SUMMARY OF THE INVENTION

In order to provide oligonucleotide probes which are as useful in PCR as in other amplification technologies such as SDA, the present invention relates to oligonucleotide primers for the amplification of a nucleic acid target sequence which is characteristic of eubacteria. These primers include SEQ ID NO:1 and SEQ ID NO:2, and can be utilized as a primer set. The invention also relates to an oligonucleotide probe which includes SEQ ID NO:3 and is used for detection of a nucleic acid target sequence which is characteristic of eubacteria.

The primer set and probe can also be used in a method for determining the presence or absence of a nucleic acid target sequence characteristic of eubacteria wherein a nucleic acid sequence amplification procedure is performed with the primer set, and the presence or absence of the nucleic acid target sequence is determined by the hybridization or lack of hybridization of the probe thereto. In order to render the method convenient for the practitioner, the present invention also encompasses a kit which includes the primer set and the probe of the present invention.

### DETAILED DESCRIPTION OF THE INVENTION

In order to aid in the understanding of the present invention, the following terms have the following meanings as used herein.

A "probe" is a synthetic or biologically produced oligonucleotide which contains specific nucleotide sequences which allow the probe to hybridize to target nucleic acid sequences. In addition to their hybridization properties, probes may also contain certain constituents that pertain to their proper optimal functioning under particular assay conditions. Such modifications are elaborations on the basic probe function which is its ability to usefully discriminate between target and non-target organisms in a hybridization assay.

A "primer" is a synthetic or biologically produced oligonucleotide which by design or selection contains specific nucleotide sequences which allow it to hybridize to a section of a target nucleic acid sequence. Primers are capable of extension by polymerases or similar enzymes to hybridize to an entire target nucleic acid sequence. Primers are utilized in nucleic acid sequence amplification procedures such as polymerase chain reaction (PCR) and Strand Displacement Amplification (SDA). Certain primers, particularly those useful in SDA techniques include, in addition to the sequence capable of hybridizing to target nucleic acid, a recognition sequence for a restriction endonuclease and an arbitrary sequence upon which the polymerase or other enzyme continuing polymerase-like activity may support itself to commence its template-specific oligonucleotide synthesis.

"Hybridization" is the process by which under predetermined reaction conditions, partially or completely complementary strands of nucleic acid are allowed to come together in an anti-parallel fashion to form a double-stranded nucleic acid with specific and stable hydrogen bonds.

As stated above, the present invention relates to oligonucleotide primers and probes which are useful for the determination of the presence or absence of a nucleic acid target sequence which is characteristic of eubacteria. The method by which such a determination is made includes an amplification procedure followed by a hybridization of the probe to amplified target nucleic acid sequence. The primers of the present invention are specific for highly conserved sequences of the 16S rRNA gene of eubacteria. The probe of the present invention is specific for an internal consensus sequence within the primer amplified product.

Bacterial 16S rRNA is known to have highly conserved sequences within its structure. Noller, H.F., and Woese, C.R., Science 212, 403 (1981); Pace, N.R., Bacteriol. Rev. 37, 562 (1973); and Woese, C.R., et al, Microbiol. Rev. 47, 621 (1983). Therefore, in order to develop the primers of the present invention, the nucleotide sequences of the 16S rRNA gene for Escherichia coli, Bacteroides fragilis, Neisseria gonorrhoeae, Staphylococcus aureus, Enterococcus and Listeria monocytogenes as identified by GenBank were aligned using Macintosh Genework's software (Intellengenetics). These 16S rRNA genes were then evaluated for highly conserved consensus sequences using the same software. The assigned position numbers were based on the E. coli 16S rRNA sequence.

Using this technique two primers were developed: (1) primer 16S 895 which comprises SEQ ID NO: 1 and corresponds to the sense strand of E. coli sequence at nucleotide positions 881 through 893; and (2) primer 16S 947 comprising SEQ ID NO:2 and corresponding to the antisense strand of E. coli sequence at nucleotide positions 947 through 960. The probe for detection of the target nucleic acid sequence amplified using the primers 16S 895 and 16S 947 comprises the antisense strand of E. coli aligns at nucleotide positions 906 through 924 and is designated SEQ ID NO:3.

The primers and probes of the present invention were then synthesized using an Applied Biosystems 380B automated synthesizer from Applied Biosystems of Foster City, California using cyanoethyl phosphoramidite chemistry as recommended by the manufacturer. The primers and probes were deprotected at 50°C overnight and purified by polyacrylamide gel electrophoresis on a Bio-Rad electroelutor as recommended by the manufacturer. These techniques are known to those in the art as evidenced by Walker G.T. et al., Nuc Acids Res. 20, 1691 (1992).

The primers and probes of the present invention were then utilized in a PCR amplification procedure followed by an SDA procedure with various genomic bacterial DNA preparations. The strains of bacteria utilized in these amplification procedures are set forth in Table 1 below.

**TABLE 1**

| BACTERIUM | ATCC No. |
|---|---|
| Staphylococcus aureus | 25923 |
| Pseudomonas aeruginosa | 27853 |
| Corynebacterium xerosis | 373 |
| Escherichia coli | 11775 |
| Klebsiella pneumoniae | 13883 |
| Serratia marcensens | 8100 |
| Morganella morganii | 25830 |
| Moraxella osloensis | 9281 |
| Haemophilus influenzae | 33533 |
| Streptococcus pneumoniae | 6303 |
| Enterobacter aerogenes | 13048 |
| Neisseria gonorrhoeae | 19424 |
| Acinetobacter lwoffi | 19001 |
| Eubacterium lentum | 43055 |
| Proteus vulgaris | 13315 |
| Streptococcus faecalis | 29212 |
| Streptococcus pyogenes | 19615 |
| Listeria monocytogenes | 7644 |

Genomic DNA from the above bacteria was prepared as described in Current Protocols in Molecular Biology, 1987, Greene Publishing Associates and Wiley-Interscience, N.Y.

### PCR Amplification of a Target Sequence of the Bacterial 16S rRNA Gene

The PCR amplification procedure was carried out in its preferred embodiment as an automated process utilizing a thermostable enzyme. In this process the reaction mixture was cycled through a denaturing step, a primer annealing step and a synthesis step. A DNA thermocycler (Perkin-Elmer) specifically adapted for use with the thermostable enzyme was utilized.

The primers of the present invention (comprising SEQ ID NO: 1 and SEQ ID NO:2) were evaluated in PCR amplification procedures using the genomic DNA of the above-identified 18 bacterial species as template and one non-bacterial genomic DNA as an additional template. The non-bacterial genomic DNA was that from Candida albicans (ATCC 44808). This fungal genomic DNA was also prepared as described in Current Protocols in Molecular Biology.

Each PCR amplification procedure was performed as a 50 µl reaction containing 100 ng of genomic bacterial or fungal DNA, 10 mM Tris-HCl pH8.3, 50 mM KC1, 1.5mM MgCl₂, 0.01% (w/v) gelatin, 200 µM each dATP, dTTP, dGTP, dCTP and 20 pmoles of each primer (comprising SEQ ID NO: 1 and SEQ ID NO:2). The reactions were overlaid with mineral oil and heated to 95°C for 5 minutes to denature target DNA followed with the addition of 1.2 units AmpliTaq® polymerase (Perkin-Elmer Cetus). Amplification parameters were 94°C for 1 minute, 37°C for 1 minute, 72°C for 2 minutes for 30 cycles followed by a 4°C soak. Detection of amplified products was determined on a 1.5% agrose gel in 1 x TBE.

The results of this PCR procedure indicated amplification of a 79 base pair fragment for all 18 bacteria tested with no cross reactivity to the Candida albicans. However, amplification of the 79 base pair fragment for Neisseria gonorrhoeae displayed a weak signal just above background.

Sequence analysis of the amplified product for Neisseria gonorrhoeae revealed two mismatches at nucleotide positions 947 and 954 of the 16 S 947 primer (SEQ ID NO:2). Therefore, the 16S 947 primer for use in the SDA procedure was designed to exclude one of these two mismatches (i.e. the mismatch at position 947). This exclusion was chosen because it was at the 5' end of the primer, whereas the other mismatch was more toward the middle of the primer. Therefore, the SDA 16S947 primer (comprising SEQ ID NO:4) only required a single base pair shift to eliminate one of the two mismatches. Also, the other mismatch (at position 954) was with the most conserved nucleotide (T).

### SDA Amplification of a Target Sequence of the Bacterial 16S rRNA Gene

Strand Displacement Amplification (SDA) is an isothermal method of nucleic acid amplification in which extension of primers, displacement of single stranded extension products, annealing of primers to the extension products (or the original target sequence) and subsequent extension of the primers occurs concurrently in the reaction mix. This is in contrast to the PCR, in which the steps of the reaction occur in discrete phases or cycles as a result of the temperature constraints of the reaction. SDA is based upon 1) the ability of a restriction endonuclease to nick the unmodified strand of a hemiphosphorothioate form of its double stranded recognition site and 2) the ability of certain polymerases to initiate replication at the nick and displace the downstream non-template strand.

After an initial incubation at increased temperature (about 95°C) to denature double stranded target sequences for annealing of the primers, subsequent polymerization and displacement of newly synthesized strands takes place at a constant temperature (usually about 37°C). Production of each new copy of the target sequence consists of five steps: 1) binding of amplification primers to an original target sequence or a displaced single-stranded extension product previously polymerized, 2) extension of the primers by exonuclease deficient (exo⁻) klenow polymerase incorporating an α-thio deoxynucleoside triphosphate, 3) nicking of a hemiphosphorothioate double stranded restriction site, 4) dissociation of the restriction enzyme from the nick site, and 5) extension from the 3' end of the nick by exo⁻ klenow with displacement of the downstream non-template strand. Nicking, polymerization and displacement occur concurrently and continuously at a constant temperature because extension from the nick regenerates another nickable restriction site.

When primers which hybridize to both strands of a double stranded target sequence are used, amplification is exponential, as the sense and antisense strands serve as templates for the opposite primer in subsequent rounds of amplification. SDA is described by G. T. Walker et al. (1922a. *Proc. Natl. Acad. Sci. USA* **89**,392-396 and 1992b. *Nuc. Acids. Res.* **20**, 1691-1696). Examples of restriction enzymes which nick their double stranded recognition sites when an α-thio dNTP is incorporated are HincII, HindII, AvaI, NciI and Fnu4HI. All of these restriction enzymes and others which display the required nicking activity are suitable for use in SDA. The Walker et al. disclosures are hereby incorporated by reference and details of the SDA method are found in the following examples.

Targets for amplification by SDA may be prepared by fragmenting larger nucleic acids by restriction with an endonuclease. However, it is most preferred that target nucleic acids are amplified without this restriction step prior to SDA through use of a target generation step. See Walker et al. (1992b) *supra*. This target generation scheme is also described in United States Patent Application Serial No. 07/794,399, filed November 19, 1991, the disclosure of which is hereby incorporated by reference. This method for generation of SDA-amplifiable target sequences comprises heat denaturing double stranded nucleic acids containing the target sequence and hybridizing four primers to the target sequence. Two of the primers (S₁ and S₂) are SDA amplification primers as defined below, with target binding sequences near their 3' ends and restriction enzyme recognition sites 5' to the target binding sequences. When both amplification primers are used amplification is exponential, however, use of only one amplification primer results in linear amplification of the target sequence. The other two primers (B₁ and B₂) are external primers as defined below and consist only of target binding sequences. S₁ and S₂ bind to opposite strands of double stranded nucleic acids flanking the target sequence. B₁ and B₂ bind to the target sequence 5' (i.e., upstream) of S₁ and S₂, respectively. Exonuclease deficient klenow polymerase (exo⁻klenow polymerase) is then used to simultaneously extend all four primers in the presence of three deoxynucleoside triphosphates and one modified deoxynucleoside triphosphate (e.g., deoxyadenosine 5'-[α-thio]triphosphate dATP[αS]). Extension of S₁ and S₂ produces two extension products, S₁-ext and S₂-ext. Extension of B₁ and B₂ results in displacement of the downstream S₁ and S₂ extension products from the original target sequence template. The displaced, single stranded S1 extension product serves as a target for binding of S₂ and B₂. Similarly, the displaced, single stranded S₂ extension product serves as a target for binding of S₁ and B₁. All four primers are then extended on the S₁ -ext and S₂-ext templates to produce a second pair of extension products which are displaced by extension of the external primers as before. Binding and extension of complementary amplification primers on these displaced extension products results in synthesis of a complementary strand. This produces two double stranded nucleic acid fragments with hemimodified restriction enzyme recognition sites at each end which are suitable for amplification by SDA. The extended external primers hybridized to S1 -ext and S2-ext form two larger double stranded fragments with hemimodified restriction enzyme recognition sites at only one end. As in SDA, the individual steps of the target generation reaction occur concurrently and continuously, generating target sequences with the required recognition sequences at the ends for nicking by the restriction enzyme in SDA. As all of the components of the SDA reaction are already present in the target generation reaction, target sequences generated automatically and continuously enter the SDA cycle and are amplified. This process is well-known to those skilled in the art from the publication of Walker G.T. et al., Proc. Nat'l. Acad. Sci. USA 89,392 (1992).

The SDA amplification primers were used to evaluate 6 ng (1 x 10⁶ genomes) of target genomic DNA from the following organisms from Table 1: Listeria monocytogenes, Eubacterium lentum, Streptococcus pyogenes, Klebsiella pneumoniae, Haemophilus influenzae, Neisseria gonorrhoeae, Staphylococcus aureus, Corynebacterium xerosis, Pseudomonas aeruginosa, and Escherichia coli. As with the PCR procedure, 6ng (1x10⁶ genomes) of genomic DNA of Candida albicans was also included in the protocol.

Each SDA reaction consisted of 6 ng target DNA, 10 ng human placental DNA, 50 mM KᵢPO₄ pH 7.4, 100 ug/ml acetylated bovine serum albumin, 7 mM MgCl₂, 23% glycerol, 0.2 mM each dCTP, dGTP, thio-dATP and 0.5 mM dUTP, 500 nM 16S895 primer (S1 primer comprising SEQ ID NO: 1 plus a HincII restriction site and additional nucleotides; this S1 primer is designated SEQ ID NO: 7) and the modified 16S947 primer (i.e. with the exclusion of the G at position 947) (S2 primer comprising SEQ ID NO:4 plus a HincII restriction site and additional nucleotides; this S2 primer is designated SEQ ID NO: 8). Also included in each reaction were external primers (B1 comprising SEQ ID NO: 5 and B2 comprising SEQ ID NO: 6) which are used to aid in creating templates for amplification. These purposes and techniques are known to those skilled in the art from publications such as Walker, G T et al., Nuc. Acids. Res. 20, 1691 (1992).

The reactions were incubated at 95°C for 3 minutes, followed by 40°C for 2 minutes. Then, 150 units of HincII endonuclease (New England Biolabs) and 2 units of exo-klenow polymerase (USB) were added to each reaction allowing amplification to occur for 2 hours at 40°C. Reactions were terminated by heating to 95°C for 3 minutes. More detailed descriptions of the techniques involved in the SDA process are known to those skilled in the art from publications such as Walker et al., Proc. Natl. Acad. Sci. USA 89, 392 (1992).

The amplified SDA products were detected using the 5'-³²P labeled detector probe (comprising SEQ ID NO:3) discussed above. The probe was kinased in a 10 ul reaction containing 1µm SEQ ID NO:3, 50 mM Tris-HCl pH 8.0, 10 mM MgCl₂, 10 Units T4 polynucleotide kinase, and 70 uCi-³²P-ATP (3000 Ci/mmol, 10 mCi/mL). The reaction was carried out at 37°C for 30 minutes and terminated by heating to 70°C for 2 minutes. SDA products were detected by primer extension in a reaction containing 0.1 µM of ³²P-detector probe in 50 mM KᵢPO₄, 0.5 mM dUTP, 0.2 mM each thio-dATP, dCTP, dGTP, 100 ug/ml BSA, 6 mM MgCl₂ and 5 µl of a SDA reaction as described by Walker, G.T. et al., Nuc. Acids Res. 20 1691(1992). Each sample was heated to 95°C for 2 minutes, and cooled to 37°C for 2 minutes. Primer extension was carried out with the addition of 2 units of exo-klenow and incubated for an additional 10 minutes at 37°C. The reactions were terminated by the addition of 7.5 µl of 50% (W/V) urea, 20 mM Na₂EDTA, 0. 5xTBE, 0.05% bromophenol blue/xylene cylanol. SDA primer extension products were analyzed by gel electrophoresis using an 8% denaturing gel and visualized by X-ray film (FUJI) exposed for 16 hours with intensifying screens at -70°C.

Target specific SDA products were observed as a ³²P-47-mer representing the HincII nicked products and a ³²P-71-mer representing the unnicked strands. These products were detected for all bacterial organisms tested, however the Eubacterium lentum signal was lower by comparison. Additionally, Candida albicans and the zero target sample with 10 ng human DNA indicated no background amplification.

To determine the sensitivity of the 16S rRNA SDA amplification primers S₁ (SEQ ID NO: 7) and S₂ (SEQ ID NO: 8), 1 to 10,000 Streptococcus pyogenes genome copies were each amplified and detected as previously outlined. Each sample also included 0.45 ug of human DNA. A 16 hour autoradiography exposure detected 10 genomic copies of Streptococcus pyogenes with no detectable background in the zero target sample.

### EXAMPLE 1

### Determination of Consensus 16S Sequences for Possible Use as Eubacterial Primers and Probes

Determination of consensus 16S sequences for possible use as eubacterial primers and probes was done by formatted alignments from Gen Bank using MACINTOSH's Gene Work program. The nucleotide sequences of the 16s rRNA gene for Escherichia coli, Bacteroides fragilis, Neisseria gonorrhoeae, Staphylococcus aureus, Enterococcus and Listeria monocytogenes as known from Gen Bank were aligned.

For the development of eubacterial primers, the 16s rRNA genes of the indicated bacteria were evaluated for areas of highly conserved consensus sequences. Two areas in the 16s rRNA sequence were chosen for primer development because of the conserved nature of the sequence at nucleotide positions 881-893 and nucleotide positions 947-960. Primer 16s895 represents the sense strand of E. coli sequence at nucleotide positions 881-893 (5'-CCTGGGGAGTACG-3') (SEQ ID NO: 1) and primer 16s947 corresponds to the antisense strand at nucleotide positions 947-960 (5'-AATTAAACCACATG-3') (SEQ ID NO: 2). These primers were initially evaluated in a PCR amplification system using 18 bacterial and 1 nonbacterial genomic DNAs as the template. PCR amplification was used to determine if the primers would detect and amplify the bacterial genomic DNA.

### EXAMPLE 2

### Polymerase Chain Reaction to Determine Effectiveness of Primers and Probes

This experiment examined the effectiveness of primers 16s895 (SEQ ID NO: 1) and 16s947 (SEQ ID NO: 2) to amplify 18 bacterial and 1 nonbacterial genomic DNAs.

The polymerase chain reaction amplification was performed in a 50 µl reaction volume. The final concentration of all the reaction components was as follows:
100 ng genomic DNA
10 mM Tris-HCL pH 8.3
50 mM KCL
1.5 mM MgCl₂
0.01% w/v gelatin
200 uM dATP
200 uM dTTP
200 uM dGTP
200 uM dCTP
20 pmole 16s895 (SEQ ID NO: 1)
20 pmole 16s947 (SEQ ID NO: 2)
The reactions were overlaid with mineral oil and heated to 95°C for 5 minutes to denature target DNA. An aliquot of AmpliTag polymerase (1.2 Units) (Perkin Elmer Cetus) was added to each reaction mixture. Amplification parameters were 94°C for 1 minute, 37°C for 1 minute, 72°C for 2 minutes for 30 cycles followed by a 4°C soak, using a Perkin Elmer DNA Thermal Cycler. Amplified products were visualized by ethidium bromide after electrophoresing over a 1.5% agarose gel in 1X TBE.

PCR results indicated amplification of a 79 bp fragment (observed strong band on 1.5% agarose gel) for all bacteria tested which included:

### STRAINS OF BACTERIA TESTED IN PCR (tested positive for the above results).

Staphylococcus aureus
Pseudomonas aeruginosa
Corynebacterium xerosis
Escherichia coli
Klebsiella pneumoniae
Serratia marcescens
Morganella morganii
Haemophilus influenzae
Streptococcus pneumoniae
Enterobacter aerogenes
Neisseria gonorrhoeae
Acinetobacter lwoffi
Eubacterium lentum
Listeria monocytogenes
Proteus vulgaris
Streptococcus faecalis
Streptococcus pyogenes
Moraxella osloensis

### NONBACTERIA TESTED IN PCR (tested negative for the above results)

### Candida albicans

Amplification of the 79 bp fragment for Neisseria gonorrhoeae indicated a weaker signal just above background. Sequence analysis for this species was shown to include two mismatches at nucleotide positions 947 and 954. For this reason, the SDA primers were modified to exclude the mismatch at position 947, and to include 3 additional nucleotides at the 3' terminus. This SDA primer represented 16s rRNA nucleotide positions 948-963 (compared to nucleotide positions 947-960 for the PCR primers).

### EXAMPLE 3

### Strand Displacement Amplification to Determine Effectiveness of Primers and Probes

This experiment examined the effectiveness of S1 (SEQ ID NO: 7) and S2 (SEQ ID NO: 8) (amplification primers)/B1 (SEQ ID NO: 5) and B2 (SEQ ID NO: 6) (outside primers) and detector probe to amplify and detect 10 bacterial and 1 nonbacterial genomic DNAs using Strand Displacement Amplification.
The Strand Displacement Amplification was performed as follows:
The SDA eubacterial primer set (amplification primers, SEQ ID NOS: 7 and 8; outside primers, SEQ ID NOS: 5 and 6) was used to evaluate 6 ng (1X10⁶ genomes) of target genomic DNA. The target DNAs were diluted on 50 mM KᵢPO₄, pH 7.4 containing 1 ng/µl human placental DNA.

The final concentration of all the reaction components and the order of addition was as follows:
50mM KᵢPO₄ pH 7.4
100µg/ml acetylated bovine serum albumin
0.2 mM each dAₛTP, dCTP, dGTP
0.5 mM dUTP
500 nM primers S1 and S2 (amplification primers, SEQ ID NOS: 7 and 8)
50 nM primers B1 and B2 (outside primers, SEQ ID NOS: 5 and 6)
7 mM MgCl₂
23% glycerol
6 ng target DNA
10 ng human placental DNA
The reactions were incubated at 95°C for 3 minutes to denature the target DNA followed by 40°C for 2 minutes to anneal the primers. Aliquots of Hinc II (150 units) and exo-klenow (USB) (2 units) were added to each reaction allowing amplification to occur for 2 hours at 40°C. Reactions were terminated by heating to 95°C for 3 minutes.

The amplified SDA products were detected using a 5'-³²P labelled detector probe (SEQ ID NO: 3). The kinase reaction to ³²P label the detector probe was performed in a 10 µl reaction volume. The final concentration of all the reaction components was as follows:
1 uM SEQ ID NO: 3
50 mM Tris-HCl pH 8.0
10 mM MgCl₂
70 uCi-³²P-ATP (3000 Ci/mmol, 10 mCi/ml)
10 units T4 polynucleotide kinase (NE Biolabs)
The reaction was carried out at 37°C for 30 minutes and terminated by heating to 70°C for 2 minutes.

The SDA products were detected in a primer extension reaction containing the kinased ³²P labelled detector probe. The reaction was performed in a 10 µl reaction volume. The final concentration of all the reaction components was as follows:
50 mM KᵢPO₄
0.5 mM dUTP
0.2 mM each dAₛTP, dCTP and dGTP
100 µg/ml acetylated bovine serum albumin
6 mM MgCl₂
0.1uM ³²P-detector probe
5 µl of an SDA reaction
Each sample was heated to 95°C for 2 minutes, and cooled to 37°C for 2 minutes. Primer extension was carried out with the addition of 2 units of exo-klenow and incubated for an additional 10 minutes at 37°C. The reactions were terminated by the addition of 7.5 µl of 50% (w/v) urea, 20 mM Na₂EDTA, 0.5X TBE, 0.05% bromophenol blue/xylene cylanol. SDA primer extension products were analyzed by gel electrophoresis using an 8% denaturing gel and visualized by x-ray film (FUJI) exposed for 16 hours with intensifying screens at -70°C-

Results: Target specific SDA products were observed as a ³²P-47-mer representing the HincII nicked products and a ³²P-71-mer representing the unnicked strands. These products were detected for all bacterial organisms tested, however the Eubacterium lentum signal was lower by comparison.

### STRAINS OF BACTERIA TESTED BY SDA (positive for the above results)

Listeria monocytogenes
Eubacterium lentum
Streptococcus pyogenes
Klebsiella pneumoniae
Haemophilus influeneae
Neisseria gonorrhoeae
Streptococcus aureus
Corynebacterium xerosis
Pseudomonas aeruginosa
Escherichia coli

### NONBACTERIA TESTED BY SDA (negative for the above results)

### Candida albicans

Although the foregoing invention has been described in detail for the purpose of illustration, it will be obvious that changes and modifications may be practiced within the scope of the appended claims by those of ordinary skill in the art.

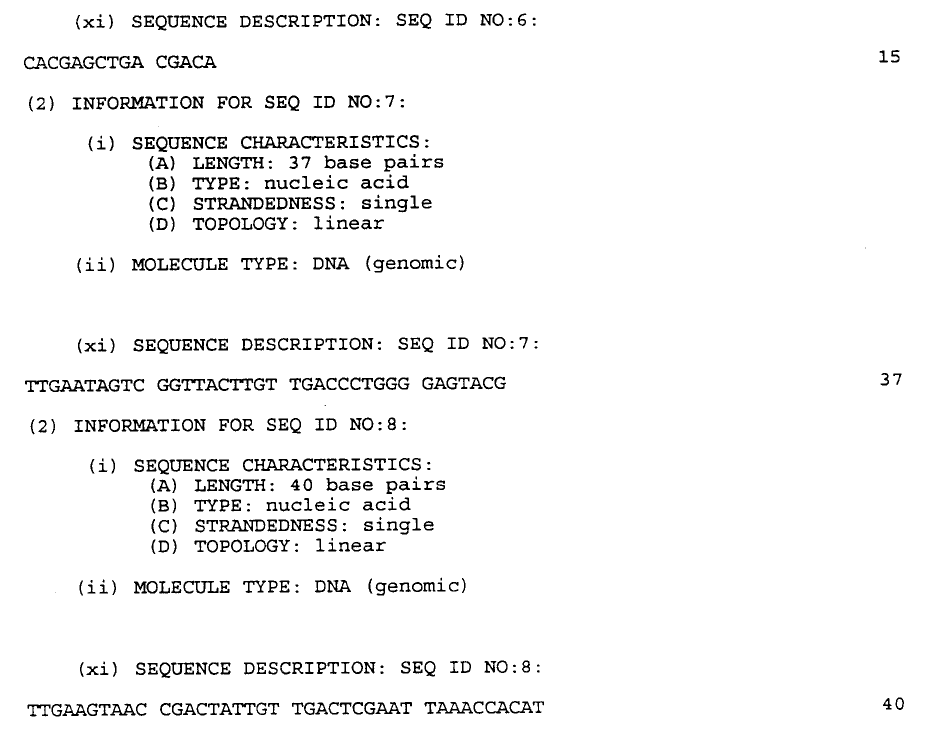

SEQUENCE LISTING

(1) GENERAL INFORMATION:

(i) APPLICANT: BECTON, DICKINSON AND COMPANY

(A) NAME: BECTON, DICKINSON AND COMPANY

(B) STREET: 1 Becton Drive
(C) CITY: Franklin Lakes
(D) STATE: NJ
(E) COUNTRY: US
(F) ZIP: 07417

(ii) TITLE OF INVENTION: Oligonucleotide Primers and Probes for
Detection of Bacteria

(iii) NUMBER OF SEQUENCES: 8

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(v) CURRENT APPLICATION DATA:
(A) APPLICATION NUMBER:
(B) FILING DATE:

(2) INFORMATION FOR SEQ ID NO:1:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:1:

CCTGGGGAGT ACG 13

(2) INFORMATION FOR SEQ ID NO:2:

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:2:

AATTAAACCA CATG 14

(2) INFORMATION FOR SEQ ID NO:3:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:3:

CGTCAATTCA TTTGAGTTT 19

(2) INFORMATION FOR SEQ ID NO:4:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:4:

TCGAATTAAA CCACAT 16

(2) INFORMATION FOR SEQ ID NO:5:

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:5:

GATTAGATAC CCTG 14

(2) INFORMATION FOR SEQ ID NO:6:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: DNA (genomic)

## Claims

1. An oligonucleotide comprising SEQ ID NO: 1.

2. An oligonucleotide comprising SEQ ID NO:2.

3. An oligonucleotide comprising SEQ ID NO: 4.

4. An oligonucleotide probe for detection of a nucleic acid target sequence which is characteristic of eubacteria, said probe comprising SEQ ID NO: 3.

5. A method for determining the presence or absence of a nucleic acid target sequence which is characteristic of eubacteria, said method comprising the steps of:
(a) performing a nucleic acid sequence amplification procedure using a primer set comprising the oligonucleotide of claim 1 and the oligonucleotide of claim 2; and
(b) determining whether the oligonucleotide probe of claim 4 hybridizes to the nucleic acid target sequence.

6. An oligonucleotide primer comprising SEQ ID NO:7.

7. An oligonucleotide primer comprising SEQ ID NO: 8.

8. A method for determining the presence or absence of a nucleic acid target sequence which is characteristic of eubacteria, said method comprising the steps of:
(a) performing a nucleic acid sequence amplification procedure using a primer set comprising the oligonucleotide primer of claim 6 and the oligonucleotide primer of claim 7; and
(b) determining whether the oligonucleotide probe of claim 4 hybridizes to the nucleic acid target sequence.

9. The method of claim 8 wherein the nucleic acid sequence amplification procedure is Strand Displacement Amplification (SDA).

10. The method of claim 9 wherein the performance of the nucleic acid sequence amplification procedure includes the use of a first external primer comprising SEQ ID NO:5 and a second external primer comprising SEQ ID NO:6.
